# EUROPÄISCHE PATENTSCHRIFT

(11) **EP 0 728 452 B1**
(45) Veröffentlichungstag und Bekanntmachung des Hinweises auf die Patenterteilung: **07.11.2001**
(21) Anmeldenummer: 96102257.1
(22) Anmeldetag: 15.02.1996
(51) Int. Cl.: A61F 5/058

(54) **Clavicula-Bandage**
Clavicle bandage
Bandage claviculaire

(30) Priorität: 21.02.1995 DE 19505854
(43) Veröffentlichungstag der Anmeldung: 28.08.1996
(73) Patentinhaber: SCHÜTT & GRUNDEI ORTHOPÄDIETECHNIK GmbH, D-23556 Lübeck (DE)
(72) Erfinder: Grundei, Hans, Dr., D-23556 Lübeck (DE)
(74) Vertreter: Fuchs Mehler Weiss & Fritzsche

(56) Entgegenhaltungen:
- GB-A- 223 577
- US-A- 1 755 641
- US-A- 3 382 868
- US-A- 3 897 776
- US-A- 4 589 406

## Beschreibung

Die Erfindung betrifft eine Clavicula-Bandage mit den Merkmalen gemäß dem Oberbegriff des Anspruchs 1, wie sie beispielsweise bekannt ist aus der US-A-3 382 868.

Dementsprechend weist die Clavicula-Bandage zwei in ihrer Länge verstellbare Bänder aus im wesentlichen nicht dehnbarem Material auf, die an jeweils ihrem ersten Ende an einem gemeinsamen Halteelement dauerhaft fixiert und mit ihrem jeweils anderen zweiten Ende an dem Halteelement lösbar fixierbar ist.

Derartige Bandagen dienen zur Therapie einer Fraktur des Schlüsselbeines. Dabei ist es ein vorrangiges Ziel, das Schlüsselbein ruhig zu stellen, damit die Frakturstellen gegeneinander möglichst nicht bewegt werden während der Heilungsphase. Selbstverständlich ist ein weiterer Aspekt bei derartigen Bandagen, daß der Tragekomfort möglichst hoch ist.

Bei der aus der gattungsbildenden Druckschrift bekannten Clavicula-Bandage besteht das Halteelement aus einem länglichem Gewebestreifen, an dem die beiden Bänder im unteren Bereich mit ihren zweiten Enden unabhängig voneinander befestigt sind, und zwar mittels jeweils eines Hakens, der in einen mit dem Halteelement verbundenen Ring greift. Hierdurch wird eine gewisse Verschwenkbarkeit der unteren Enden der Bänder erzielt, und zwar um den Mittelpunkt des Ringes. Dies gestattet ein Anpassen der Clavicula-Bandage bei Bewegungen des Schulterblattes in gewissen Grenzen. Nicht ausgeschlossen werden kann allerdings, daß die unteren Endbereiche der Bänder auf den unteren Bereich der Schulterblätter drücken, was den Tragekomfort und damit die Akzeptanz dieser Bandage negativ beeinflußt.

Aus der EP-A-0 379 929 ist eine weitere Clavicula-Bandage bekannt, bei der in Abweichung von der gattungsgemäßen Clavicula-Bandage das Halteelement aus einem Ring besteht, an dem die beiden Bänder wiederum mit ihren zweiten Enden unabhängig voneinander befestigt sind, wobei darauf Wert gelegt wird, daß die Bänder über diesen Ring zueinander winkelverschieblich verbunden sind. Die Winkelverschieblichkeit soll bei einer hohen Therapiesicherheit, d.h. bei einem anatomisch guten Sitz ohne Verrutschen und Lockern einfach anzulegen und für den Patienten angenehm zu tragen sein und darüberhinaus leicht nachstellbar sein.

Dadurch, daß beide Bänder quasi punktförmig an dem auf dem Rücken des Patienten zu liegen kommenden Ring anfangen und nach erfolgtem Anlegen dort wieder enden, indem nämlich die Verschlüsse, welche sich an den jeweils anderen Enden der Bänder befinden, an dem Ring geschlossen werden, ist dieser Rückenteil relativ hohen Belastungen, d. h. Zugkräften und infolgedessen einem hohen Druck ausgesetzt, was den Tragekomfort entgegen den in der besagten Druckschrift gemachten Ausführungen herabsetzt. Darüber hinaus wird der natürliche Bewegungsablauf durch die bekannte Clavicula-Bandage nicht optimal unterstützt.

Schließlich sei noch auf die US-A- 5 074 292 hingewiesen, aus welcher unter anderem eine Anwendung einer relativ großen Pelotte als Teil einer Clavicula-Bandage bekannt ist. Die Pelotte überdeckt ganze Teile des Rückens eines Patienten und somit auch die beweglichen Schulterblätter. Der Tragekomfort dieser Clavicula-Bandage genügt daher in keiner Weise den Erwartungen der Patienten.

Vor dem aufgezeigten Hintergrund ist es die Aufgabe der vorliegenden Erfindung, die eingangs erwähnte gattungsgemäße Clavicula-Bandage dahingehend zu verbessern, daß einerseits eine größtmöglichste Bewegungsfreiheit für den Patienten erhalten bleibt und andererseits, daß das frakturierte Schlüsselbein unter Zug gebracht wird unter gleichzeitiger möglichst effektiver Ruhigstellung der Frakturstelle. Darüberhinaus soll die Clavicula-Bandage patientenindividuell eingestellt werden können im Hinblick auf eine erfolgreiche Therapie.

Gelöst wird diese Aufgabe dadurch, daß erfindungsgemäß das Halteelement im oberen Bereich eine Lochleiste aufweist, mittels derer die beiden ersten Enden mit Befestigungsmitteln höhenverstellbar an dem Halteelement fixiert sind, und daß das Halteelement als längliches Plattenelement im wesentlichen T-förmig ausgebildet ist, wobei der große Schenkel von kaudal nach kranial verläuft.

Die fest fixierten Enden der beiden Bänder können dann patientenindividuell in der jeweiligen orthopädischen Werkstatt auf die wirksame Länge des Halteelements eingestellt werden. Damit können verschiedene Körpergrößen der Patienten berücksichtigt werden, wenn die Clavicula-Bandage als Universalteil nur in einer Größe konfektioniert wird. Die Enden der Bänder können in den jeweiligen Löchern beispielsweise mit Nieten dauerhaft fixiert werden.

Durch die Ausbildung des Halteelementes als längliches Plattenelement wird -wie schon im Falle der gattungsbildenden Druckschrift- ein gewisser "Rucksackeffekt" erzielt. Im Unterschied zu der gattungsgemäßen Clavicula-Bandage ist das Halteelement jedoch -wie angegeben- T-förmig ausgebildet. Der Querschenkel des T-förmigen Halteteils liegt unterhalb der Schulterblätter am Rücken des Patienten an. Die spezielle Ausbildung des Halteelements ermöglicht es dem Patienten, daß beispielsweise beim Gehen die Arme mitbewegt werden können, wobei diese Bewegung sich über die Schulterblätter bis auf den hinteren mittleren bis unteren Bereich des Rückens auswirkt, ohne daß das Halteelement diese Bewegung störend beeinflussen würde. Auf diese Art und Weise wird der größtmögliche Tragekomfort einer Clavicula-Bandage erzielt.

Gemäß einer bevorzugten Ausführungsform gehen die beiden dauerhaft fixierten Enden beider Bänder vom Halteelement so ab, daß die Hauptachse des Halteelementes mit den Bändern nach oben hin jeweils einen Winkel im Bereich von 40° - 50° einschließt. Diese Ausbildung ergibt eine anatomiegerechte Einleitung der Zugkräfte auf den Patientenkörper, läßt es aber andererseits auch zu, daß die Schlüsselbeinfraktur, die meist in etwa der Mitte des Schlüsselbeins auftritt, frei von Beeinträchtigungen der Bänder bleibt.

Aus Symmetriegründen liegen die Fixationspunkte der ersten, dauerhaft fixierten Enden der Bänder und der Drehpunkt beider Schnallen für die zweiten Enden der Bänder vorzugsweise auf der Hauptachse des Halteelementes. Hierdurch wird eine weitgehende Kraftsymmetrie erreicht, gleiche Bänderspannung vorausgesetzt.

Schließlich besteht das Halteelement vorzugsweise aus zähelastischem Plastikmaterial, welches angenehm zu tragen ist.

Weitere Merkmale in konkreten Ausführungsbeispielen können realisiert sein. Beispielsweise sollten die Bänder aus atmungsaktiven Material bestehen, wie etwa Baumwolle etc..

Die Erfindung wird anhand eines Ausführungsbeispieles näher erläutert. Hierbei zeigt:
- Fig. 1: eine Ansicht einer Clavicula-Bandage von hinten, und
- Fig. 2: eine vergrößerte Darstellung des Halteelementes.

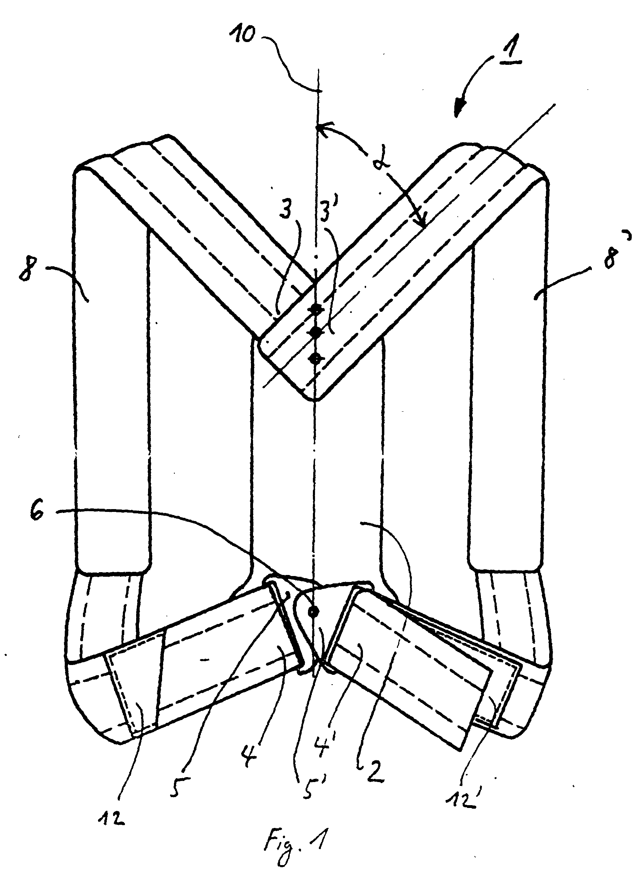

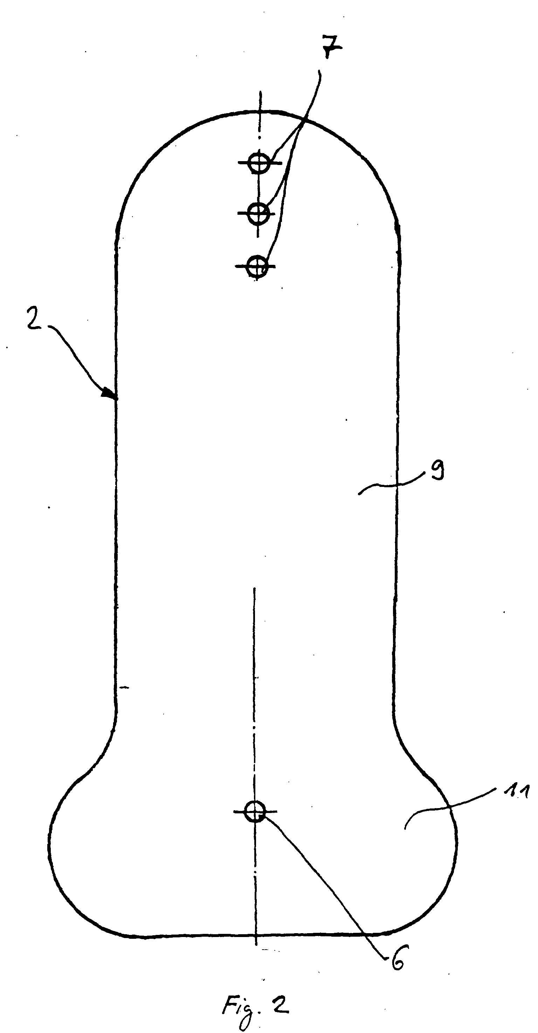

Deutlich erkennbar in Fig. 1 sind die beiden Bänder 8 und 8' der Clavicula-Bandage 1. Die Bänder weisen jeweils ein erstes Ende 3 bzw. 3' und ein zweites Ende 4 bzw. 4' auf. Die ersten Enden 3 und 3' sind im oberen Bereich des als längliches Plattenelement ausgebildeten Halteelements 2 dauerhaft fixiert, beispielsweise mit (nicht dargestellten) Nieten, die durch einige Löcher der Lochleiste 7 (Fig. 2) geführt sind. Dies gestattet es, daß die Clavicula-Bandage in der orthopädischen Werkstatt patientenindividuell eingestellt werden kann, d. h. die wirksame Länge des Halteelementes wird durch geeignete Auswahl der Löcher, durch die Nieten hindurch gesetzt werden, festgelegt.

Gezeigt ist hier im übrigen auch der Winkel α zwischen den beiden Bändern 8 und der Hauptachse 10 der Clavicula-Bandage, der vorliegend im Bereich zwischen 40° - 50° liegt.

Das Halteelement 2 ist als T-förmiges Teil ausgebildet, dessen langer Schenkel 9 (Fig. 2) nach oben weist, also überhalb der Wirbelsäule am Rücken zu liegen kommt und von kaudal nach kranial verläuft.

Etwa im Schnittpunkt der Hauptachse 10 des Halteelementes 2 mit der Achse seines Querschenkels 11 ist ein Drehpunkt 6 in Form einer Bohrung vorgesehen. An diesem Drehpunkt 6 sind beispielsweise durch eine Niete zwei Schnallen 5 und 5' verschwenkbar angelenkt. Die Schnallen 5 und 5' dienen zur Aufnahme der zweiten Enden 4 und 4' der Bänder 8 bzw. 8'. Die Enden 4 und 4' werden durch die Schnallen hindurchgeführt und beispielsweise mit einem Klettenverschluß 12 und 12' an der Schnalle 5 bzw. 5' fixiert.

Während des Gehens paßt sich die Clavicula-Bandage 1 durch die Mitbewegung der Schulterblätter dem Bewegungsvorgang an, indem nämlich die Schnallen 5 und 5' eine gewisse Mitbewegung durch die Verschwenkung um den Drehpunkt 6 erfähren, ohne allerdings dadurch die Ruhestellung des Schlüsselbeines zu beeinträchtigen. Ein wesentlich erhöhter Tragekomfort ist die Folge, was wiederum die Akzeptanz bei den Patienten fördert und dies wiederum zu einem besseren Therapieerfolg führen kann.

## Patentansprüche

1. Clavicula-Orthese mit länglichem Halteelement (2) und mit zwei in ihrer Länge verstellbaren Bändern (8,8') aus im wesentlichen nicht dehnbarem Material, die an jeweils ihrem ersten Ende (3,3') im oberen Bereich des gemeinsamen Halteelementes (2) dauerhaft fixiert und mit ihrem jeweils anderen zweiten Ende (4,4') im unteren Bereich des Halteelements (2) mittels zweier Schnallen (5,5') lösbar so fixiert sind, daß die Schnallen um einen Drehpunkt (6) verschwenkbar sind,
**dadurch gekennzeichnet,**
**daß** das Halteelement (2) im oberen Bereich eine Lochleiste (7) aufweist, mittels derer die beiden ersten Enden 4, 4' mit Befestigungsmitteln höhenverstellbar an dem Halteelement (2) fixiert sind, und
**daß** das Halteelement (2) als längliches Plattenelement im wesentlichen T-förmig ausgebildet ist, wobei der große Schenkel (9) von kaudal nach kranial verläuft.

2. Clavicula-Bandage nach Anspruch 1, **dadurch gekennzeichnet, daß** die beiden ersten Enden (3, 3') beider Bänder (8, 8') so vom Halteelement (2) abgehen, daß die Hauptachse (10) des Halteelementes (2) mit den Bändern (8, 8') nach oben hin jeweils einen Winkel α im Bereich von 40° < α < 50° einschließt.

3. Clavicula-Bandage nach Anspruch 1, **dadurch gekennzeichnet, daß** die Fixationspunkte der ersten Enden (3, 3') und der Drehpunkt (6) beider Schnallen (5, 5') auf der Hauptachse (10) des Halteelementes (2) liegen.

4. Clavicula-Bandage nach einem der Ansprüche 1 bis 3, **dadurch gekennzeichnet, daß** das Halteelement (2) aus zähelastischem Plastikmaterial besteht.

## Claims

1. Clavicle orthotic device having elongated support element (2) and having two tapes (8, 8'), which can be adjusted in their length, made from essentially non-elastic material, which are permanently fixed to in each case their first end (3, 3') in the upper region of the common support element (2) and releaseably fixed to their in each case other second end (4, 4') in the lower region of the support element (2) by means of two buckles (5, 5'), so that the buckles can be pivoted around a pivot (6), **characterised in that** the support element (2) has in the upper region a punched strip (7), by means of which the two first ends (3, 3') are fixed by fastening means in height-adjustable manner to the support element (2), and **in that** the support element (2) as an elongated plate element is designed to be essentially T-shaped, wherein the large leg (9) runs caudally to cranially.

2. Clavicle bandage according to claim 1, **characterised in that** the two first ends (3, 3') of both tapes (8, 8') branch off from the support element (2), so that the main axis (10) of the support element (2) with the tapes (8, 8') encloses upwards in each case an angle α in the range from 40° < α < 50°.

3. Clavicle bandage according to claim 1, **characterised in that** the fixation points of the first ends (3, 3') and the pivot (6) of both buckles (5, 5') lie on the main axis (10) of the support element (2).

4. Clavicle bandage according to one of claims 1 to 3, **characterised in that** the support element (2) consists of viscoplastic plastic material.

## Revendications

1. Orthèse claviculaire comprenant un élément de maintien (2) longitudinal et deux bandes (8, 8') réglables en longueur, réalisées dans un matériau sensiblement non extensible, dont la première extrémité (3, 3') de chacune est fixée de manière permanente contre la partie supérieure de l'élément de maintien (2) commun et dont la deuxième extrémité (4, 4') de chacune est fixée de manière amovible, au moyen de deux boucles (5, 5'), contre la partie inférieure de l'élément de maintien (2), de telle sorte que les boucles peuvent pivoter autour d'un point de rotation (6),
**caractérisée en ce que** l'élément de maintien (2) comporte dans la partie supérieure un rebord perforé (7), au moyen duquel les deux premières extrémités (4, 4')¹ sont fixées avec des moyens de fixation contre l'élément de maintien (2) de manière à pouvoir régler leur hauteur, et **en ce que** l'élément de maintien (2) est conçu sous forme d'élément plat longitudinal sensiblement en forme de T, dans lequel la grande branche (9) s'étend de la région caudale vers la région cervicale.

2. Orthèse claviculaire selon la revendication 1, **caractérisée en ce que** les deux premières extrémités (3, 3') des deux bandes (8, 8') partent de l'élément de maintien (2), de telle sorte que l'axe principal (10) de l'élément de maintien (2) forme vers le haut avec chacune des bandes (8, 8') un angle α compris dans une plage de valeurs de 40°< α < 50°.

3. Orthèse claviculaire selon la revendication 1, **caractérisée en ce que** les points de fixation des premières extrémités (3, 3') et le point de rotation (6) des deux boucles (5, 5') sont situés sur l'axe principal (10) de l'élément de maintien (2).

4. Orthèse claviculaire selon l'une quelconque des revendications 1 à 3, **caractérisée en ce que** l'élément de maintien (2) est réalisé dans une matière plastique viscoélastique.
